# EUROPEAN PATENT APPLICATION

(11) **EP 0 692 481 A1**
(43) Date of publication of application: **17.01.1996**
(21) Application number: 95906529.3
(22) Date of filing: 27.01.1995
(51) Int. Cl.: C07D 323/06

(54) **PROCESS FOR PRODUCING TRIOXANE**

(30) Priority: 31.01.1994 JP 9582/94
(71) Applicant: POLYPLASTICS CO. LTD., Chuo-Ku Osaka-shi Osaka 541 (JP)
(72) Inventor: KASHIHARA, Osamu, Shizuoka 416 (JP); KUROISHI, Takeo, Shizuoka 417 (JP); HIRAGOORI, Motohito, Shizuoka 417 (JP); AKIYAMA, Minoru, Shizuoka 417 (JP); KASAI, Yuzo, Shizuoka 416 (JP); FUKUI, Yuichi, Shizuoka 416 (JP)
(74) Representative: Jackson, Peter
(86) International application number: JP9500098
(87) International publication number: WO9520586

(57) **Abstract**

A process for producing trioxane from an aqueous formaldehyde solution at a high distillation efficiency in an energy-saving manner, which comprises the following steps (i) to (iv): (i) the step of forming trioxane by passing a highly concentrated aqueous solution of formaldehyde through a reactor packed with a solid acid catalyst; (ii) the step of extracting the formed trioxane from the aqueous formaldehyde solution containing the same with a water-insoluble organic solvent having a boiling point higher than that of trioxane and incapable of forming an azeotrope therewith; (iii) the step of removing low-boiling substances contained in the organic solvent phase containing trioxane by distillation or evaporation; and (iv) the step of distilling the trioxane-containing organic solvent freed from the low-boiling substances, thereby obtaining trioxane from the top of the distillation column and returning the organic solvent from the bottom of the column to the step (ii).

## Description

### Field of the art

The present invention relates to a process for preparing trioxane as the starting material of polyoxymethylene from an aqueous solution of formaldehyde, and more particularly provides a process for preparing trioxane according to which process energy can be consistently saved in preparation of trioxane from an aqueous solution of formaldehyde. Arts in the background

In general, a process for preparing trioxane from an aqueous solution of formaldehyde comprises the following three operations:
(1) an operation of synthesizing trioxane from an aqueous solution of formaldehyde,
(2) an operation of separating the resulting aqueous solution of formaldehyde containing trioxane obtained through synthesis thereof from a catalyst, and
(3) an operation of separating and purifying trioxane from the aqueous solution of formaldehyde containing trioxane.

Accordingly, in order to drastically save energy during the course of preparation of trioxane from an aqueous solution of formaldehyde, novel techniques consistent through these operations are necessary.

In a conventional process for preparing pure trioxane as has heretofore been employed, a 30 to 70 wt. % aqueous solution of formaldehyde is heated and distilled in the presence of a liquid acid such as sulfuric acid to synthesize trioxane while withdrawing the resulting aqueous solution of formaldehyde containing formed trioxane out of the reaction system to thereby obtain distillate hazing a composition comprising 20 to 55 wt. % of trioxane, 17 to 35 wt. % of formaldehyde and 20 to 50 wt. % of water, and trioxane is then extracted from the distillate with a water-insoluble or difficultly water-soluble solvent to obtain an extraction liquor, which is rectified to separate therefrom trioxane (Japanese Patent Publication No. 6,344/1966).

Further, processes wherein a variety of solid acid is used as a catalyst for synthesis instead of the liquid acid in the foregoing process are disclosed, for example, in Japanese Patent Publication No. 12,794/1965, Japanese Patent Laid-Open No. 263,985/1983, and Japanese Patent Laid-Open No. 134,786/1984.

There have been proposed various solvents for use in extraction of trioxane from an aqueous solution of formaldehyde containing trioxane and withdrawn out of a reaction system through distillation or evaporation thereof (Japanese Patent Publication No. 6,344/1966 and Japanese Patent Publication No. 12,671/1967).

Further, Japanese Patent Laid-Open No. 145,485/1991 discloses a process for preparing trioxane, wherein a 30 to 85 wt. % aqueous solution of formaldehyde is fed to a distillation column, a liquid withdrawn from the bottom of the column is flowed into a reactor packed with an acid catalyst, especially a solid acid catalyst, and the resulting aqueous solution of formaldehyde containing trioxane flowed out of the reactor is circulated into an intermediate tray portion of the distillation column.

In all of these processes, however, a large amount of energy is necessary because after synthesis of trioxane from an aqueous solution of formaldehyde in the presence of an acid catalyst the resulting aqueous solution of formaldehyde containing trioxane is withdrawn out of the reaction system and concentration of trioxane, if necessary, is effected through distillation or evaporation.

More specifically, when withdrawal of trioxane out of the reaction system is effected through distillation or evaporation, inclusion of a large amount of water into a vapor phase (distillate) enriched with trioxane cannot be avoided in association with vapor-liquid equilibrium between trioxane, formaldehyde and water as also described in the aforementioned Japanese Patent Publication No 6,344/1966, and evaporation of this water entails loss of a large amount of heat of vaporization (sensible heat and latent heat), thus increasing the amount of energy necessary for preparation of trioxane.

On the other hand, in Japanese Patent Laid-Open No. 49,250/1992, there has been proposed a process wherein a step of effecting a reaction in the presence of a solid acid catalyst is combined with a step of extraction to extract trioxane from a liquid reaction mixture. According to this process, at least two reactors packed with a solid acid are provided, at least one reactor thereof is used to effect a reaction while forcibly circulating an aqueous solution of formaldehyde or a liquid mixture of an aqueous solution of formaldehyde and an extractant to thereby synthesize trioxane, the liquid reaction mixture is brought into contact with the extractant to extract and separate therefrom trioxane, and the resulting aqueous solution of formaldehyde after the extraction of trioxane is used as the starting material for synthesis of trioxane in other reactor. In this process, however, at least two reactors are indispensable, and a number of extractors corresponding to the number of the reactors are necessary or use of a single extractor (column) unavoidably makes the configuration thereof complicated, thus necessitating a huge cost of installation of reaction facilities. Where the aqueous solution of formaldehyde containing synthesized trioxane is simultaneously brought into contact with the extractant and the solid acid catalyst, long-term use of the catalyst results in deterioration thereof due to a reaction caused between them, and hence involves a fear of lowering the reaction yield. Further, according to the process as disclosed in this patent document wherein the aqueous solution of formaldehyde lowered in formaldehyde concentration after synthesis of trioxane in the first reactor and subsequent extraction thereof in the extractor is fed to another reactor to synthesize trioxane and the foregoing procedure is sequentially repeated, the equilibrium of the reaction of formaldehyde to trioxane in the second and following, if any, reactor(s) is lowered in sequence to lower the efficiency of extraction and make later isolation of trioxane difficult despite an improvement in the apparent conversion of formaldehyde as the starting material to trioxane because the synthesis reaction of formaldehyde to trioxane is an equilibrium reaction. Particularly where trioxane is extracted using an organic solvent lower in boiling point than trioxane as described in Example of Japanese Patent Laid-Open No. 49,250/1992, it is anticipated that a huge amount of energy will he consumed in later operations of separating and purifying trioxane because of the above-mentioned lowering of the extraction efficiency resulting from the lowering of the equilibrium of the reaction of formaldehyde to trioxane.

As described hereinbefore, in the conventional processes for preparing trioxane, no drastic attempts to save energy consumed during the course of preparation of trioxane by a consistent process ranging from synthesis of trioxane to purification of trioxane in preparation of trioxane from an aqueous solution of formaldehyde have been made with no technological ideas concerning a consistent drastic energy-saving measure.

In view of the foregoing circumstances, the inventors of the present invention have proposed in EP-A 583 907 corresponding to Japanese Patent Application No. 208,265/1992 a process for preparing pure trioxane from an aqueous solution of formaldehyde, wherein trioxane is synthesized and withdrawn out of the reaction system without involving a change in the phase of water contained therein, and wherein a choice is made of an organic solvent suitable for purification of trioxane from the reaction system. According to this process, a considerable amount of energy can be saved in preparation of trioxane. As a result of further investigations, however, it has been found out that in this process a minute amount of a low-boiling component including water and formaldehyde is contained in an extraction liquor resulting from extraction of trioxane into an organic solvent from an aqueous solution of formaldehyde containing trioxane to present a problem of lowering the distillation efficiency in subsequent purification of trioxane.

### Brief Description of the Drawings

Fig. 1 is a diagram showing an example of a process flow suitable for carrying out the process for preparing trioxane according to the present invention.

Fig. 2 is a diagram showing an example of a process flow wherein the step of removing a low-boiling component is omitted from the process for preparing trioxane according to the present invention.

Fig. 3 is a diagram showing an example of a process flow for carrying out a conventional process for preparing trioxane.
1 represents evaporator, 2 represents reactor, 3 represents extractor, 4 represents evaporator, 5 represents distillation column, 6 represents reactor, 7 represents distillation column.

### Disclosure of the Invention

In view of the above, the inventors of the present invention have made intensive investigations with a view to further improving the technology of EP-A 583 907 corresponding to Japanese Patent Application No. 208,265/1992 wherein the distillation efficiency of the extractant phase left intact is low because it still contains the minute amount of the low-boiling component including water and formaldehyde. As a result, the present invention has been completed.

Specifically, the present invention provides a process for preparing trioxane from an aqueous solution of formaldehyde, characterized by comprising the following steps (i) to (iv) of:
(i) pasting a high-concentration aqueous solution of formaldehyde through a reactor packed with a solid acid catalyst to obtain trioxane;
(ii) extracting trioxane from the resulting aqueous solution of formaldehyde containing trioxane obtained in the step (i) with a water-insoluble organic solvent having a higher boiling point than trioxane and having no azeotropic composition thereof with trioxane;
(iii) preliminarily removing a low-boiling substance contained in the resulting organic solvent phase containing trioxane and obtained in the step (ii) through distillation or evaporation thereof; and
(iv) subjecting the resulting trioxane-organic solvent system stripped of the low-boiling component in the step (iii) to a distillation operation to obtain trioxane from the top of a distillation column while returning the organic solvent from the bottom of the distillation column to the step (ii).

The process of the present invention is first of all characterized in that it does not involve an operation of concentrating trioxane by firing an aqueous solution of formaldehyde containing trioxane in withdrawal from the reaction system of trioxane synthesized from an aqueous solution of formaldehyde as can be seen in conventional technologies, e.g., the aforementioned processes disclosed in Japanese Patent Publication No. 6,344/1966 and Japanese Patent Laid-Open No. 145,485/1991. More specifically, trioxane synthesized is separated from the aqueous solution of formaldehyde as the starting material without involving a change in the phase of water according to the process of the present invention, whereby not such a large amount of energy involved in evaporation of water as can be seen in the conventional technologies is necessary. Secondly, such a complicated operation as proposed in the process disclosed in Japanese Patent Laid-Open No. 49,250/1992 is unnecessary, and feeding of the always-controlled high-concentration aqueous solution of formaldehyde into the reactor can maintain the equilibrium concentration of trioxane in the liquid reaction mixture at a high level to efficiently effect extraction of trioxane from the liquid reaction mixture and subsequent separation and purification thereof. Thirdly, in the process of the present invention, the extractant is chosen while paying attention to rationalization of the operations of separation and purification of trioxane as well after extraction of trioxane. Thus, there has been provided a process for preparing trioxane wherein all steps ranging from the step of synthesizing trioxane from the aqueous solution of formaldehyde to the step of purifying trioxane can be made efficient consistently to realize saving of a great amount of energy. Fourthly, preliminary removal of the minute amount of the low-boiling component including water and formaldehyde as has been problematic in the invention of EP-A 583 907 can make the trioxane purification operation efficient.

A detailed description will now be made of the process for preparing trioxane according to the present invention.

First, the high-concentration aqueous solution of formaldehyde to be used in the present invention has a concentration (in terms of formaldehyde) of 40 to 85 wt. %. Herein, when the concentration of the high-concentration aqueous solution of formaldehyde is lower than 40 wt. %, the conversion of formaldehyde to trioxane in the step (i) is poor. When it exceeds 85 wt. %, paraform is formed to make the operations difficult. It had better be adjusted to be preferably 55 to 80 wt. %, further preferably 60 to 75 wt. %.

The above-mentioned high-concentration aqueous solution of formaldehyde is prepared using as the starting material an aqueous solution of formaldehyde obtained by a customary method of preparing formaldehyde, such as a methanol oxidation method (either a so-called excess air method or a so-called excess methanol method) or a methylal oxidation method (e.g., Japanese Patent Laid-Open No. 287,051/1989), or formaldehyde gas. It is obtained by concentrating or diluting the starting material by a customary operation such as absorption, evaporation or distillation. More specifically, (1) where the formaldehyde concentration of the aqueous solution of formaldehyde as the starting material is below a desired concentration, it will suffice to concentrate the solution to the desired concentration by an evaporation or distillation operation. On the other hand, (2) where the aqueous solution of formaldehyde as the starting material exceeds the desired concentration or where formaldehyde gas is used as the starting material, it will suffice to add thereto water or an aqueous solution of formaldehyde to secure the desired concentration.

The step (i) is a step of passing a high-concentration aqueous solution of formaldehyde through a reactor containing a solid acid catalyst to yield trioxane.

The solid acid catalyst to be used herein may he either an organic solid acid or an inorganic solid acid, or a mixture of at least 2 kinds of such acids. Examples of the organic solid acid include ion exchange resins, ion exchange membranes, and ion exchange fibers each having sulfonic groups, fluoroalkanesulfonic groups, or the like. On the other hand, examples of the inorganic solid acid include acid clay; silica; alumina; inorganic matter-oxide composites such as silica-alumina, alumina-boria, and zeolite; and solid carriers impregnated with sulfuric acid, phosphoric acid, boric acid or the like. The body of the reactor containing the solid acid catalyst may be of any type, examples of which include a packed bed type, a pipe type, a cage type, a fluidized bed type, and a tray column type. Various conditions involved in the reaction, e.g., the kind of catalyst, the amount of the catalyst to be packed in the reactor, the amount of the aqueous solution of formaldehyde to be fed as the starting material, the residence time, and the reaction temperature, are interrelated with one another, and hence cannot be determined univocally. In general, however, these conditions are set in such a way that the reaction of formaldehyde to trioxane proceeds so sufficiently in the reactor that the trioxane concentration at the outlet of the reactor can become substantially the equilibrium concentration for the aqueous solution of formaldehyde as the starting material. In setting these conditions, consideration is given to the reactivity and heat resistance of the catalyst, the throughput, and the capacity of the reactor as well as the practicability thereof. Among these various conditions, the reaction temperature is generally preferably 85 to 130°C. When it is lower than 80°C, the reaction rate is slow. When it exceeds 130°C, a Cannizzaro reaction as a side reaction is increased. As for the residence time in the reactor, other conditions are preferably set in such a way that it become usually 25 seconds to 25 minutes. When the residence time is shorter than 25 seconds, the reaction does not sufficiently proceeds or the control of the reaction becomes difficult even if a catalyst high in reactivity is chosen. On the other hand, when it exceeds 25 minutes, the Cannizzaro reaction as the side reaction is increased and a large reactor becomes necessary to entail a lack of practicability though it depends on a desired throughput.

Next, the step (ii) is a step of feeding the aqueous solution of formaldehyde containing trioxane obtained in the step (i) into an extractor without a change in the phase thereof to extract trioxane with a water-insoluble organic solvent having a higher boiling point than trioxane and having no azeotropic composition thereof with trioxane. The organic solvent to be used herein is required to have a higher boiling point than trioxane, to have no azeotropic composition thereof with trioxane, and to be insoluble in water. Further, the density of the organic solvent is preferably 0.8 to 0.95 or 1.05 to 1.15, and the trioxane partition coefficient of the organic solvent to the aqueous solution of formaldehyde is preferably at least 0.5.

The use of the solvent higher in boiling point than trioxane is because it is advantageous in an aspect of energy efficiency to distil out trioxane as the smaller-amount component on the top side of the column (as a vapor phase) in the distillation operation after extraction. Further, the use of the solvent having no azeotropic composition thereof with trioxane is necessary for avoiding complicated operations after distillation. Further, the density of the organic solvent is specified in the above-mentioned preferred range from the viewpoint of facilitating liquid dispersion and phase dispersion in the extraction operation. Further, the partition coefficient is specified in the preferred range of at least 0.5 from the viewpoint of securing at least a given extraction efficiency.

Preferable examples of the organic solvent endowed with the foregoing various characteristics include saturated aliphatic hydrocarbons, halogenated aliphatic hydrocarbons, alicyclic hydrocarbons, aromatic hydrocarbons, alkylated aromatic compounds, and halogenated aromatic compounds. Especially preferable specific examples of the organic solvent include nonane, 1-chlorooctane, naphthalene, phenyl ether, anisole, monochlorobenzene, dichlorobenzenes such as o-dichlorobenzene, xylenes, acetophenone, benzophenone, diethylbenzene, cumene, mesitylene, and biphenyl.

Meanwhile, the mode of extracting trioxane in the step (ii) is not particularly limited in so far as use is made of an extractor of a type as is usually used. Examples of the usable type include a packed tower type and a tray column type. There may be adopted any one of a contact mode, a counter flow mode, and a parallel flow mode. Further, the concentration of trioxane in the organic solvent after the extraction operation is usually 1 to 10 wt. % though it usually depends on the reaction equilibrium concentration depending on the concentration of the aqueous solution of formaldehyde at the time of synthesis of trioxane and the distributability of trioxane into the organic solvent.

The great feature of the present invention lies in such a combination of using as the extractant the solvent endowed with the aforementioned characteristics with feeding the aqueous solution of formaldehyde containing trioxane obtained in the reactor into the extractor without a change in the phase thereof to extract trioxane with the solvent. This greatly contributes to preparation of pure trioxane with a smaller amount of energy. Such an effect can hardly be expected either where distillation or evaporation is adopted for separation of trioxane from the reaction system, or where a solvent not endowed with the aforementioned characteristics is used for extraction of trioxane.

The step (iii), which is most characteristic of the present invention, is a step of preliminarily removing the minute amount of the low-boiling component including water and formaldehyde entrained in the extraction step from the organic solvent phase containing trioxane and obtained in the step (ii).

The content of the low-boiling component in the organic solvent phase containing trioxane is generally a few tens of ppm to 2,000 ppm. The component is removed in this step by a flash vaporization or evaporation operation. A distillation column of about 1 to a few seconds in vaporization time or an evaporator is used as a unit for that purpose. The operation temperature, though different depending on the kind of organic solvent chosen, is preferably a little lower than the boiling point of the organic solvent.

The last step (iv) is a step of subjecting the trioxane-organic solvent system not containing even the minute amount of the low-boiling component after the step (iii) to the distillation operation to obtain trioxane from the top of the distillation column while returning the organic solvent from the bottom of the distillation column to the extraction step (ii).

The type of the distillation column to be used in this step may be any one of a tray column type, a bubble-cap tower type, and a packed tower type. A variety of method can be used depending on the kind of organic solvent to be used and the trioxane concentration of the solution thereof.

The process for preparing trioxane according to the present invention enables energy to be saved consistently during the course of preparation of trioxane from an aqueous solution of formaldehyde, and especially enables a distillation operation in the course of separation and purification of trioxane to be facilitated by preliminarily removing a minute amount of a low-boiling component including water and included at the time of extraction. Further, since an organic solvent for extraction is not flowed into a reactor, deterioration of a catalyst with the organic solvent need not be considered, and a running operation of the reactor is therefore easy.

### Examples

The following Examples will specifically illustrate the present invention, but should not be construed as limiting the scope of the present invention.

### Example 1

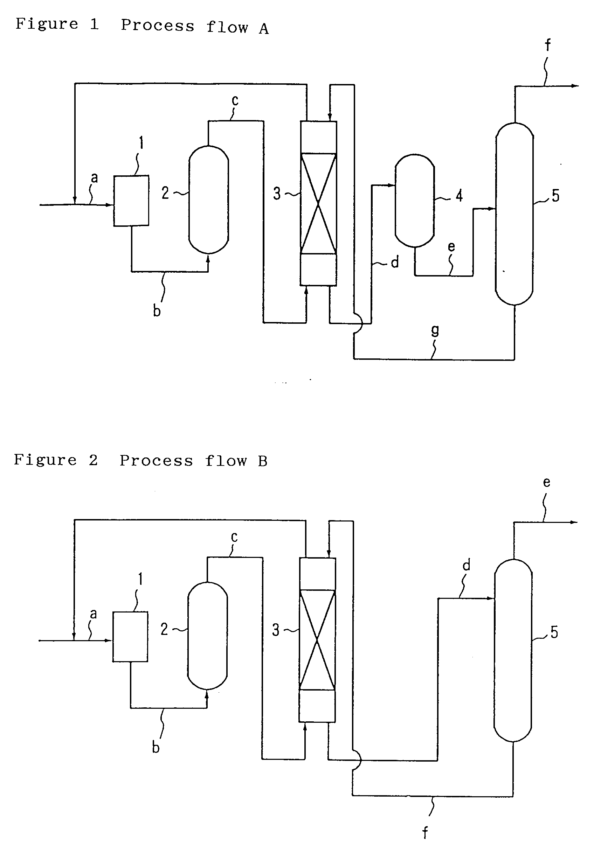

Experimental equipment was assembled in accordance with the process flow A of Fig. 1. In the figure, reference numeral 1 refers to an evaporator having a capacity of 3 liters, 2 to a reactor filled with 300 cm³ of DIAION SK104 (styrene type strongly acidic cation exchange resin manufactured by Mitsubishi Kasei Corporation), 3 to an extractor of a packed tower type having an inner diameter of 30 mm and a height of 1.5 m and filled with Raschig rings, 4 to an evaporator having a capacity of 3 liters, and 5 to a distillation column having an inner diameter of 30 mm and 40 sieve trays.

A 50 wt. % aqueous solution of formaldehyde was first fed at 100 g/hr via a line a into the evaporator 1, wherein it was combined with a solution recovered from the extractor 3 and concentrated to prepare a 60 wt. % aqueous solution of formaldehyde. The concentrated aqueous solution of formaldehyde was then passed via a line b through the reactor 2 kept at 95°C to obtain an aqueous solution of formaldehyde containing 3.5 wt. % of trioxane (step (i)).

The obtained aqueous solution of formaldehyde containing trioxane was then fed via a line c into the extractor 3, while at the same time passing therethrough o-dichlorobenzene via a line g at a flow rate of 2,600 mℓ/hr to extract trioxane from the aqueous solution of formaldehyde (step (ii)). Herein, 800 ppm of water was entrained in trioxane + o-dichlorobenzene. The extraction liquor was fed via a line d into the evaporator 4, wherein flash vaporization was effected to remove water (step (iii)). No water was contained in a solution withdrawn from the bottom of this evaporator. The resulting extraction liquor containing no water was fed via a line e into the distillation column 5, wherein a distillation operation was carried out to obtain 46 g/hr of trioxane from the top of the column (step (iv)).

In the foregoing Example, the amount of steam necessary for obtaining 1 g of trioxane was 1.87 g.

### Comparative Example 1

Experimental equipment was assembled in accordance with the process flow B of Fig. 2. In Fig. 2, reference numeral 1 refers to an evaporator having a capacity of 3 liters, 2 to a reactor filled with 300 cm³ of DIAION SK104 (styrene type strongly acidic cation exchange resin manufactured by Mitsubishi Kasei Corporation), 3 to an extractor of a packed tower type having an inner diameter of 30 mm and a height of 1.5 m and filled with Raschig rings, and 5 to a distillation column having an inner diameter of 30 mm and 20 sieve trays. In short, the evaporator 4 for flash vaporization for the purpose of removing a minute amount of a low-boiling point components after extraction was dispensed with in the process flow B of Fig. 2 in comparison with Example 1.

Like in example 1, a 50 wt. % aqueous solution of formaldehyde was fed at 100 g/hr via a line a into the evaporator 1, wherein it was combined with a recovered solution and concentrated to prepare a 60 wt. % aqueous solution of formaldehyde. The concentrated aqueous solution of formaldehyde was then passed via a line b through the reactor 2 kept at 95°C to obtain an aqueous solution of formaldehyde containing 3.5 wt. % of trioxane.

The obtained aqueous solution of formaldehyde containing trioxane was then fed via a line c into the extractor 3, while at the same time passing therethrough o-dichlorobenzene via a line f at a flow rate of 2,600 mℓ/hr to extract trioxane from the aqueous solution of formaldehyde. The resulting extraction liquor was fed via a line d into the distillation column 5, wherein a distillation operation was carried out to obtain 46 g/hr of trioxane from the top of the column. However, 0.4% of water was contained in this trioxane.

In the foregoing Comparative Example, the amount of steam necessary for obtaining 1 g of trioxane was 1.80 g.

### Examples 2 to 5

Substantially the same procedure as in Example 1 in accordance with the process flow A was repeated except that the catalyst, the organic solvent for extraction and the flow rate of the organic solvent as used in Example 1 were changed as shown in Table 1. Additionally stated, the results of Examples 2 to 5 are shown together with the results of Example 1 in Table 1.

### Comparative Examples 2 to 5

Substantially the same procedure as in Comparative Example 1 in accordance with the process flow B was repeated that use was made of the same catalyst, the same organic solvent for extraction and the same flow rate of the organic solvent as used in each Example having the same No. as that of Comparative Example, while dispensing with flash evaporation for removing a minute amount of a low-boiling component after extraction. Additionally stated, the results of Comparative Examples 2 to 5 are shown together with the results of Comparative Example 1 in Table 1.

### Comparative Example 6

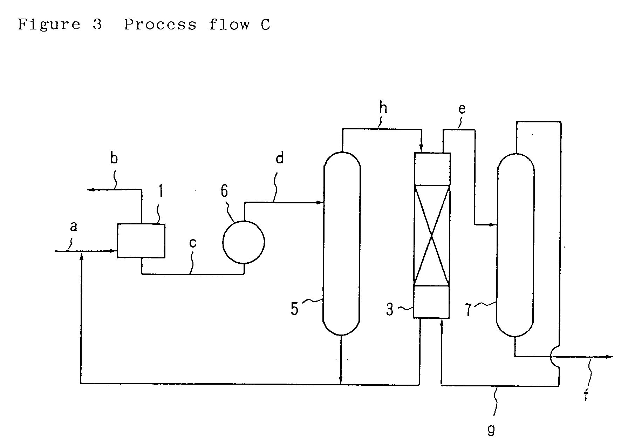

Fig. 3 shows an example of a process flow in accordance with a conventional process for preparing trioxane. In this Example, experimental equipment was assembled in accordance with the process flow C of Fig. 3, and then operated.

In Fig. 3, reference numeral 1 refers to an evaporator having a capacity of 3 liters, 6 to a evaporation-by-heating type reactor having a capacity of 5 liters and charged with 2 wt. % of sulfuric acid, 3 to an extractor of a packed tower type having an inner diameter of 30 mm and a height of 0.5 m and filled with Raschig rings, and 5 and 7 to distillation columns each having an inner diameter of 30 mm and 20 sieve trays.

A 50 wt. % aqueous solution of formaldehyde was fed at 100 g/hr via a line a into the evaporator 1, wherein it was combined with a recovered solution and concentrated to prepare a 60 wt. % aqueous solution of formaldehyde. The concentrated aqueous solution of formaldehyde was then passed via a line c through the reactor 6, wherein evaporation was effected while effecting the reaction by heating to obtain an aqueous solution of formaldehyde containing 17 wt. % of trioxane via a line d. This solution was further subjected to distillation in the distillation column 5 to obtain an aqueous solution of formaldehyde containing 41 wt. % of trioxane from the top of the column. The obtained aqueous solution of formaldehyde containing trioxane was fed via a line h into the extractor 3 while at the same time feeding benzene via a line g into the extractor 3 at a flow rate of 200 mℓ/hr to extract trioxane from the aqueous solution of formaldehyde. The resulting extraction liquor was fed via a line e into the distillation column 7, wherein a distillation operation was carried out to obtain 42 g/hr of trioxane from the bottom of the distillation column 7. No water was contained in this trioxane.

In this comparative Example, the amount of steam necessary for obtaining 1 g of trioxane was 9 g.

In the table 1,
- *1: cation exchange resin X:
a styrene type strongly acidic cation exchange resin manufactured by Mitsubishi Kasei Corporation
cation exchange resin Y:
a fluoroalkanesulfonic acid type ion exchange resin manufactured by Du Pont
- *2: naphthalene/diethylbenzene = 9:1 (weight ratio)

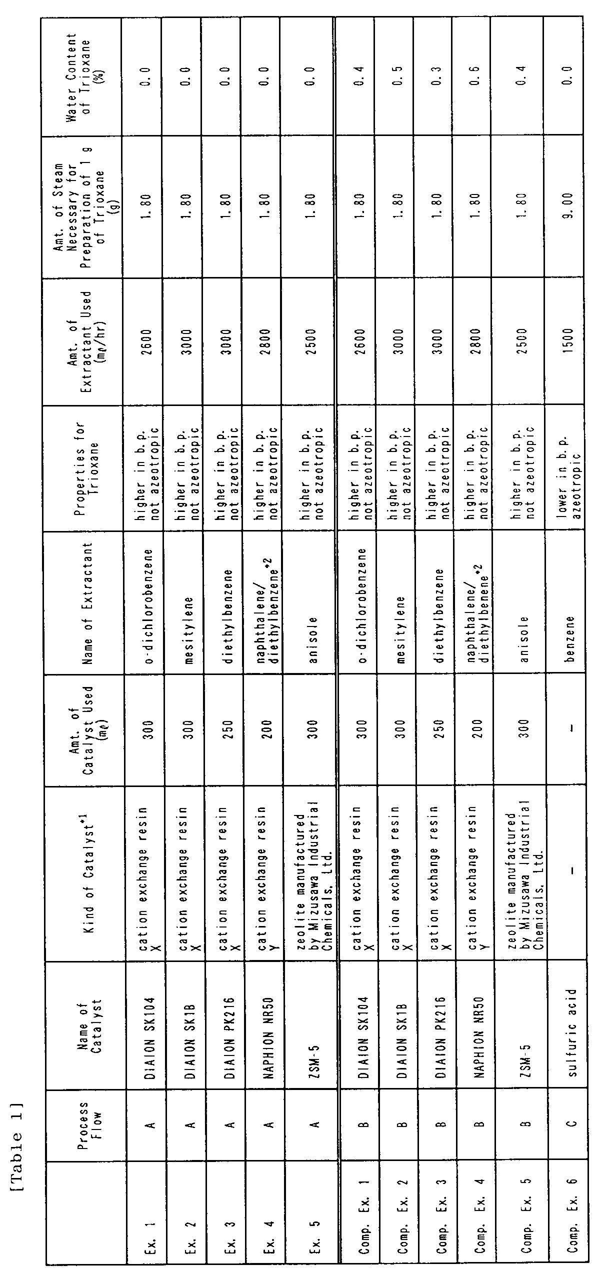

## Claims

1. A process for preparing trioxane from an aqueous solution of formaldehyde, characterized by comprising the following steps (i) to (iv) of:
(i) passing a high-concentration aqueous solution of formaldehyde through a reactor packed with a solid acid catalyst to obtain trioxane;
(ii) extracting trioxane from the resulting aqueous solution of formaldehyde containing trioxane obtained in the step (i) with a water-insoluble organic solvent having a higher boiling point than trioxane and having no azeotropic composition thereof with trioxane;
(iii) preliminarily removing a low-boiling substance contained in the resulting organic solvent phase containing trioxane and obtained in the step (ii) through distillation or evaporation thereof; and
(iv) subjecting the resulting trioxane-organic solvent system stripped of said low-boiling component in the step (iii) to a distillation operation to obtain trioxane from the top of a distillation column while returning said organic solvent from the bottom of said distillation column to the step (ii).

2. A process for preparing trioxane as claimed in claim 1, wherein the formaldehyde concentration of said high-concentration aqueous solution of formaldehyde is 40 to 85 wt.%.

3. A process for preparing trioxane as claimed in claim 1 or 2, wherein the method of obtaining said high-concentration aqueous solution of formaldehyde is either (1) a method wherein a starting aqueous solution of formaldehyde having a formaldehyde concentration lower than 40 wt.% is concentrated to a desired concentration through an evaporation or distillation operation, or (2) a method wherein a starting aqueous solution of formaldehyde having a formaldehyde concentration exceeding 85 wt.% or formaldehyde gas is admixed with water or an aqueous solution of formaldehyde to secure a desired concentration.

4. A process for preparing trioxane as claimed in claims 1, wherein said solid acid catalyst to be used is an inorganic solid acid catalyst, an organic solid acid catalyst, or a mixture thereof.

5. A process for preparing trioxane as claimed in 1, wherein said organic solvent to be used is selected from among saturated aliphatic hydrocarbons, halogenated aliphatic hydrocarbons, alicyclic hydrocarbons, aromatic hydrocarbons, alkylated aromatic compounds, halogenated aromatic hydrocarbons, aromatic ethers, and aromatic ketones, which may be used alone or in mixture.
